# EUROPEAN PATENT SPECIFICATION

(11) **EP 3 690 437 B1**
(45) Date of publication and mention of the grant of the patent: **22.03.2023**
(21) Application number: 19382081.8
(22) Date of filing: 04.02.2019
(51) Int. Cl.: G01N 33/00, G01N 33/02, F25D 17/04

(54) **GAS ABSORBING MODULE COMPRISING A GAS SENSOR CONTROL DEVICE**
GASABSORPTIONSMODUL MIT GASSENSORKONTROLLEINHEIT
MODULE D'ABSORPTION COMPRENANT UN DISPOSITIF DE CONTRÔLE D'UN CAPTEUR DE GAZ

(43) Date of publication of application: 05.08.2020
(73) Proprietor: KERACO, S.A., 22130 Peralta de Alcofea, Huesca (ES)
(72) Inventor: LATRE NAVARRO, CARLOS JOSE, 08110 MONTCADA I REIXAC (ES)
(74) Representative: Durán-Corretjer, S.L.P.

(56) References cited:
- WO-A1-2010/051735
- WO-A1-2014/143175
- CN-A- 108 802 290
- DE-A1- 10 122 306
- US-A1- 2016 195 462

## Description

The present invention relates to a gas sensor control device for use in particular in equipment for capturing gas, such as ethylene, in particular ethylene capture equipment used for the conservation of fruit and flowers.

The sensor and absorbing module according to the present invention apply to any type of gas, preferably, to hydrocarbon gases, more preferably to alkenes, still more preferably to the group of gases made up of the farnesenes, the oxidation products thereof and ethylene, and even more preferably, to ethylene.

It is known that fruits emit particular gases, such as ethylene (ethene), and other alkenes, such as α-farnesenes and the oxidation products thereof, and various pheromones.

Fruit, vegetables and flowers produce ethylene (C₂H₄) naturally, even after harvesting. The fruit ripening process begins with the production and release of said ethylene by said fruit. Once outside the fruit, the released ethylene, which is a gas, is the agent that promotes the ripening, not only of the outer skin of the fruit, but also the inner areas, since the fruit structures are permeable to said gas.

The accumulation of said gases (particularly ethylene and farnesenes) causes the accelerated ripening of said perishable products and therefore leads to a loss of quality and a reduction in the shelf life of said products. There are free pathogens in the air which affect the quality of fruit, vegetables and flowers, which in the presence of ethylene show increased proliferation causing the vegetable tissue of the perishable products to deteriorate. Moreover, in fruit and vegetable storage units, there is a constant incoming and outgoing flow of perishables, which facilitates the entry of bacteria and pathogens into the storage unit.

It is known that if the ethylene is removed from the atmosphere in which the fruit is located, the ripening process is slowed down significantly. Also known are so-called 'ethylene absorption filters', 'ethylene absorbers' or simply 'ethylene filters' which remove the ethylene from the air. Said filters normally comprise zeolite covered with potassium permanganate. Zeolite has a three-dimensional structure which maximises and increases the active surface area of the permanganate. Permanganate, in contact with ethylene, oxidises and converts said ethylene into carbon dioxide and water. It will be appreciated that any substance that oxidises ethylene by contact or captures said ethylene can be used as an ethylene filter. To increase its efficiency, said substance is usually presented in granular form.

Industrially, fruit conservation devices are found in modules or machines in storage units and/or in transport vehicles. The ethylene filter is positioned inside the module or machine. Said devices, like the air purification device disclosed in document WO 2010/051735 A1, are usually associated with a refrigeration device or air conditioning device causing the airflow produced inside the unit where the fruit is situated to pass through the device. It is also possible for the module or machine to have its own ventilators to force air to pass through the filters. Alternatively, if there is no forced airflow, the modules are positioned near the crates of fruit, preferably in the upper portion, as ethylene is slightly lighter than air and tends to rise.

A problem with these modules is that the ethylene-absorbing material (ethylene filter) has a limited life, which depends, among other things, on the amount of ethylene processed. Generally, the ethylene-absorbing material changes colour when said material loses its properties owing to oxidation, but this requires to frequently going to, and opening the module to access the material. This is problematic because, among other things as indicated above, the storage units are usually full of fruit/vegetables or flowers and because the modules or machines must be placed in locations remote from the storage unit, which makes access difficult. However, to avoid the problems associated with having ethylene in the air, the inside of the module must be accessed frequently to check the integrity of the ethylene-absorbing material.

Ethylene sensors in applications in fruit-ripening units are known. In said units, the effect sought is the opposite of that concerned in the present invention. Said units control the atmosphere contained therein and inject ethylene to accelerate the ripening of green fruit harvested, so as to be able to put the fruit quickly on the market. In general, said units usually only operate at times of peak demand.

However, a drawback of said sensors is that the useful life thereof is very short, lasting scarcely a year if used continuously. In addition, it is difficult to ascertain the precise life a particular sensor will have, because the humidity conditions, particles in the atmosphere and ethylene concentration may vary. This is an additional drawback since said sensors also require specialised knowledge for the maintenance and changing of the sensor. However, fruit storage companies are generally technologically simple enterprises which operate on low margins and do not have staff available to carry out said tasks. Consequently, the use of sensors to detect when the ethylene filters need changing causes additional problems.

An object of the present invention is to disclose means for determining when the gas (preferably ethylene) filters of the gas (preferably ethylene)-absorbing modules should be continuous which do not have the drawbacks described above.

According to the present invention an ethylene-absorbing module comprising a gas sensor control device according to independent claim 1 is provided. An object of the device is to extend the lifespan of the sensor under the atmospheric conditions normally found in fruit, vegetable and flower storage, thus facilitating maintenance.

More specifically, the present invention discloses an ethylene-absorbing module comprising a gas sensor control device for fruit conservation installations, which comprises a closed, preferably sealed, casing with a gate that can be actuated, the gas sensor being arranged inside the casing and the gate giving access to the measurement area of the sensor, the device also comprising automatic control means for the selective opening of the gate so that the sensor can measure the gas concentration. The gas is preferably a hydrocarbon, more preferably an alkene, still more preferably one of the group made up of ethylene, the farnesenes and the oxidation products thereof, and pheromones and, even more preferably, the gas is ethylene.

Said control device uses a containment unit for the sensor and automatic means for opening/closing the sensor containment unit in order to extend the useful life thereof. The device allows measurements to be carried out discontinuously at programmed intervals, while the sensor is protected from the atmosphere between measurements. In addition, it is possible to program automatic maintenance and/or recalibration tasks at times when the sensor is not taking measurements (when the gate is closed). Thus, sensors that operating under continuous measurement conditions would last a year, could see their life extended to five years. This significantly reduces the specialised maintenance required.

Preferably, the automatic means comprise a programmable electronic circuit.

More preferably, the device may comprise a screen configured to show the result of a measurement taken by the sensor. Still more preferably, said device may comprise a warning light. The warning light may give warning if the gas measurement is considered excessive. Said light may also give warning of breakdowns and/or malfunctions, or alternatively of correct operation. The screen and/or the warning light may be controlled logically from said electronic circuit.

According to the present invention, the automatic control means can activate a motor which, in turn, actuates an actuator for the gate.

In a preferred embodiment, the actuator slides along a groove in order to close/open an aperture in the gate using a closing part. According to another alternative embodiment, the actuator causes the gate to rotate so that an aperture in the gate coincides with the walls or an aperture of a housing for the gas sensor. Other embodiments are also possible.

According to the present invention, the ethylene-absorbing module for fruit conservation omprises an outer casing and a space for receiving gas filters, ethylene filters or ethylene absorbing material, and the gas sensor control device according to the present invention.

Preferably, the gate of the control device is situated inside the module.

More preferably, the module comprises means for forcing air to pass through a space intended to receive air filters, the device being situated between said space and an outlet in the module for the forced air. More preferably, the means for forcing the passage of the air comprises at least one ventilator.

Preferably, the module comprises a filter for solids situated between the space for receiving air filters and the device.

Still more preferably, the module comprises an outer casing with wheels for the transport thereof.

Advantageously, said screen and/or warning light of the device are arranged on the outside of the module.

For a better understanding, the accompanying drawings show, as an explanatory but non-limiting example, an embodiment of the present invention.
- Fig. 1 is a perspective view of an embodiment of an ethylene-absorbing module or machine according to the present invention.
- Fig. 2 is a perspective view of the module, with the front door removed.
- Fig. 3 is a perspective view of the module, exploded so that the main parts making up said module can be identified.
- Fig. 4 is a perspective view with the front removed of an embodiment of the ethylene sensor control device according to the present invention that is applied to the module shown in the previous figures.
- Fig. 5 is a perspective view of the rear portion of the device of Fig. 4.
- Fig. 6 is a perspective view of the internal components of the device, and of the rear portion of the outer casing in which said components are housed.
- Fig. 7 is a perspective view of the sensor and of the opening mechanism from inside the mechanism, and of the rear portion of the outer casing in which said mechanism and sensor are housed, with the mechanism in the closed position.
- Fig. 8 is a perspective view of the sensor and of the opening mechanism from inside the mechanism, and of the rear portion of the outer casing in which said mechanism and sensor are housed, with the mechanism in the open position.
- Fig. 9 corresponds to Fig. 7, viewed from outside the mechanism.
- Fig. 10 corresponds to Fig. 8, viewed from outside the mechanism.
- Fig. 11 is a perspective view of the rear portion of a second alternative embodiment of the device of Fig. 5.
- Fig. 12 shows the different parts separated, which show the sensor protection mechanism of the second embodiment.
- Fig. 13 shows the sensor and the motor of the second embodiment, seen from inside the device.
- Fig. 14 corresponds to the previous figure, with the sensor removed, so that the sensor reception area can be seen.
- Fig. 15 and 16 correspond to situations where the mechanism is open and closed, that is, giving access to, or closing, the sensor.

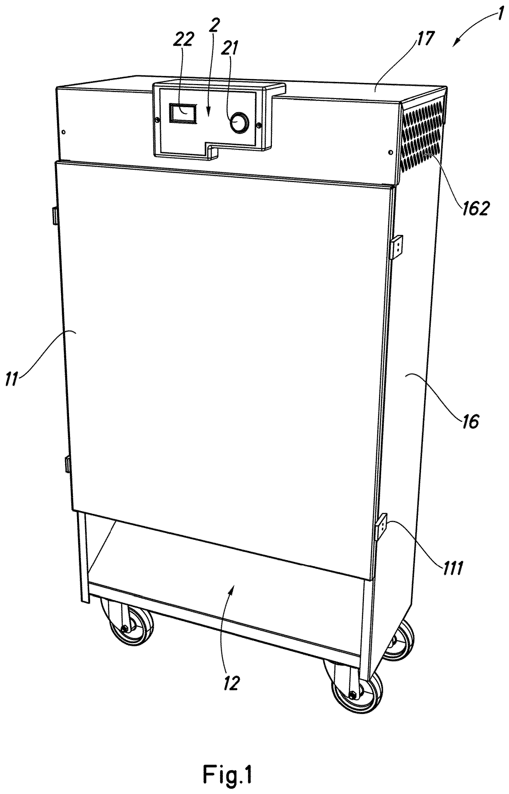

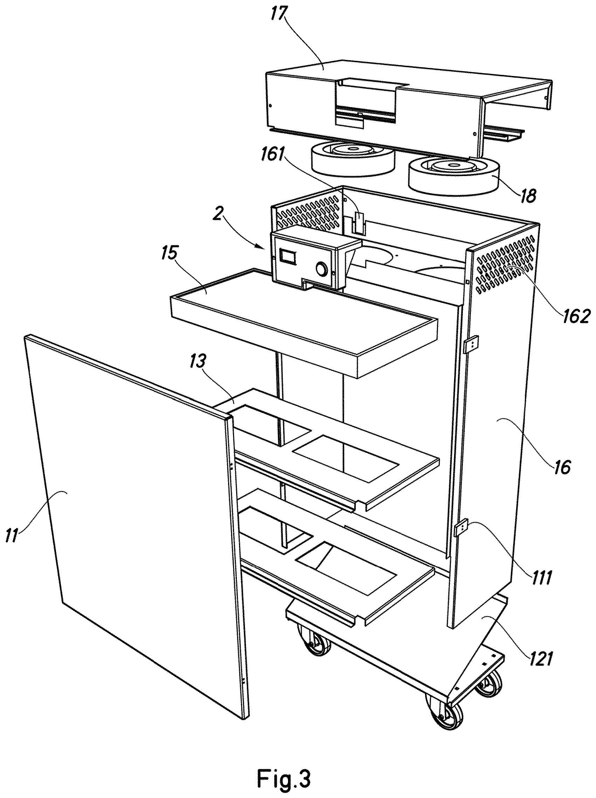

Fig. 1 to 3 show an embodiment of the ethylene-absorbing module or machine -1- according to the present invention.

Strictly speaking, a device which comprises an outer casing or cage in which a gas (for example ethylene) filter or gas (for example ethylene)-absorbing material can be positioned is usually known as a module, whereas a module with means for forcing air to pass through, such as a fan or ventilator, is known as an absorbing machine. In this application, for reasons of simplicity, the word 'module' also refers to modules provided with means for forcing air to pass through, in other words, machines.

The module -1- shown comprises a surrounding sheet metal body -16- and a closing door -11-. In this case, the surrounding body -16- has hook-and-pull closures -111- for attaching the door -11-. It will be appreciated that other closure solutions for the door -16- are also possible. At the bottom, the module -1- has an air inlet -12- formed in this case by the space between a sloping ramp -121- and the door -11-. In the case shown, the module -1- has wheels to facilitate movement. Said wheels are arranged on the part that forms the ramp -121-, being the support thereof.

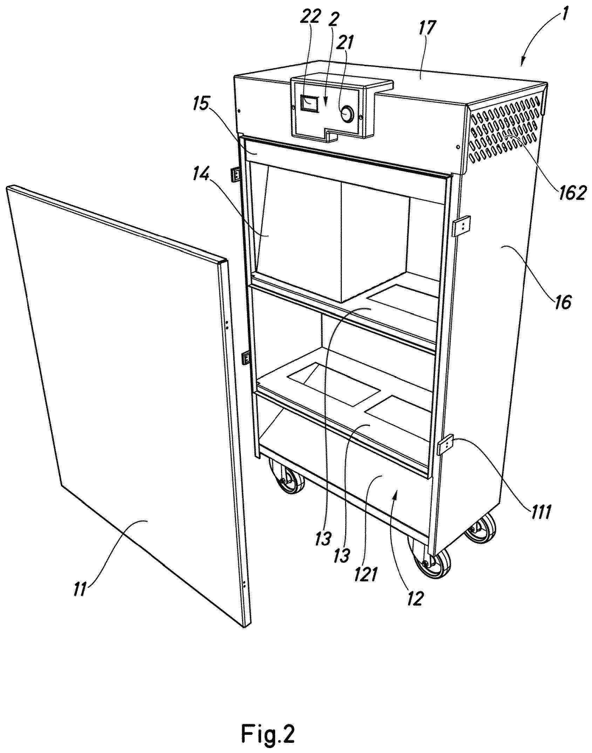

Inside, the module has shelves -13- to receive ethylene filters -14-. In Fig. 2 a single ethylene filter -14- has been shown, although it should be understood that the module shown has space for four filters. The number may vary. The filter -14- has only been shown diagrammatically. It should be understood that, in general, the filter will have a casing with a grating or slats that give access to a space in which the gas absorption/oxidation material is arranged. The filter could also simply be a porous bag with material inside. The shelves -13- in the example have apertures to make it easier for the air which is introduced through the air inlet -12- to rise towards the outlet supplied for that purpose (in this case the grating -162- situated in the upper portion of the surrounding body -16-).

Above the ethylene filter area there is a solids filter -15-. The function of this filter is to filter solid particles in the air, which could damage the ethylene sensor, before said sensor can be accessed.

In the upper portion of the module -1- shown, an ethylene sensor control device -2- is arranged, which has its own case, and some centrifugal ventilators -18-, the function of which is to create suction which causes air to enter through the inlet -12- and leave through the outlet grating -162-. A sheet metal ventilator support -17- closes the outer casing of the module -1- at the top. In the upper portion there is also an inlet -161- for electricity supply cables for actuating the ventilators -18-.

The casing of the ethylene sensor control device -2- has a screen -22- which gives the user information, for example, a quantitative measurement of the ethylene concentration detected and a warning light -21- which can give an alarm signal, for example if ethylene is detected above the set limit, or if there is a malfunction. Both the screen -22- and the warning light -21- can be seen from the outside.

The function of the sensor control device -2- is to protect the ethylene sensor, by opening and closing access to the sensor selectively to carry out measurements of the ethylene concentration at the required times, which may be pre-programmed. For example, access to the sensor will open periodically. The period may be fixed or may vary according to the concentration measured (for example, the period between measurements may depend on the last concentration measured, thus reducing the period when the concentration approaches a set limit.

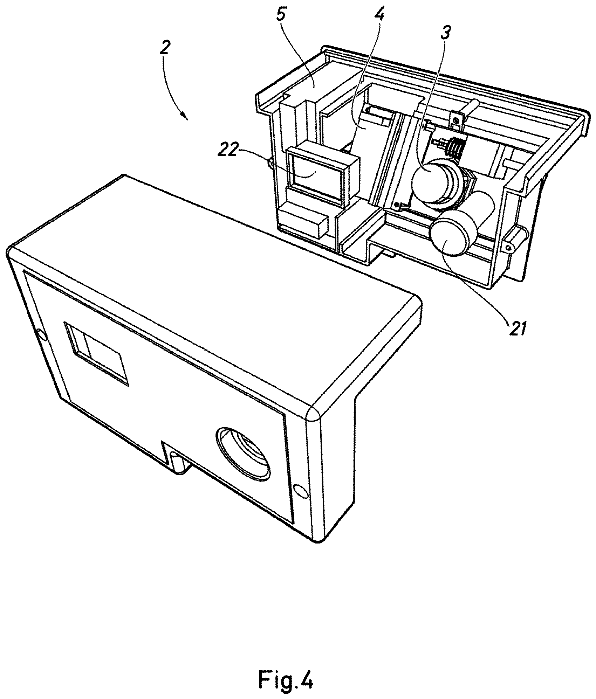

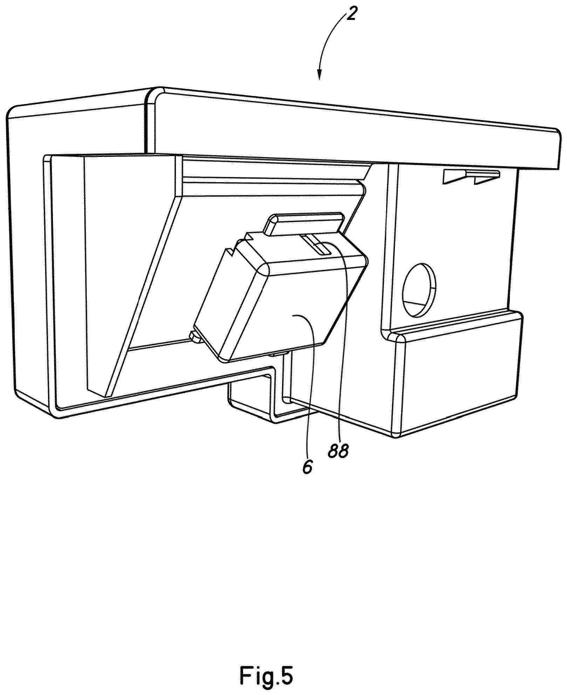

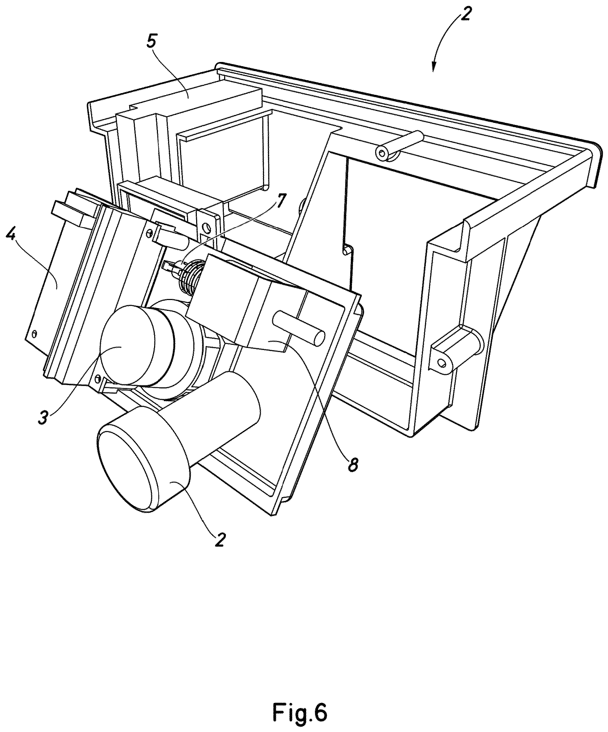

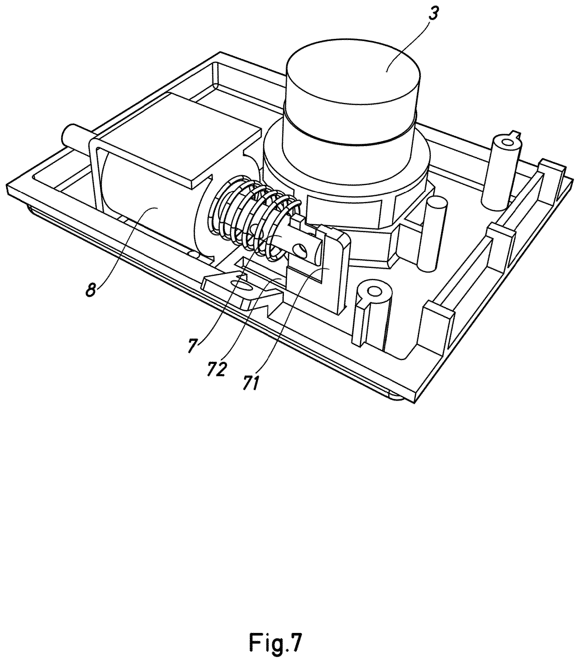

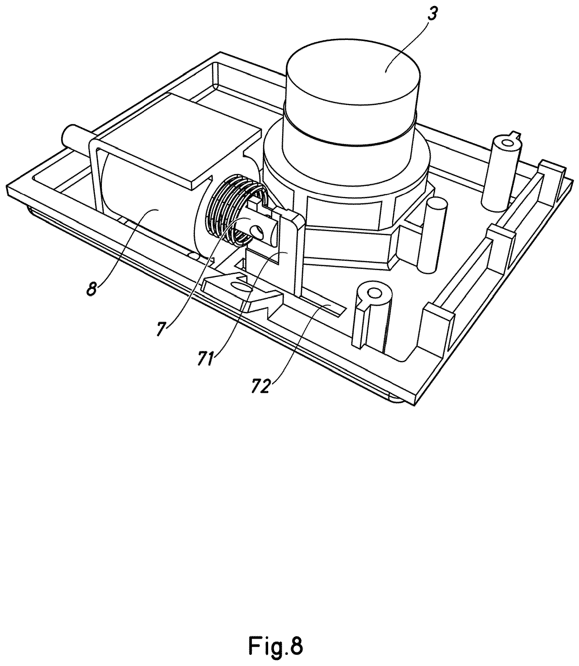

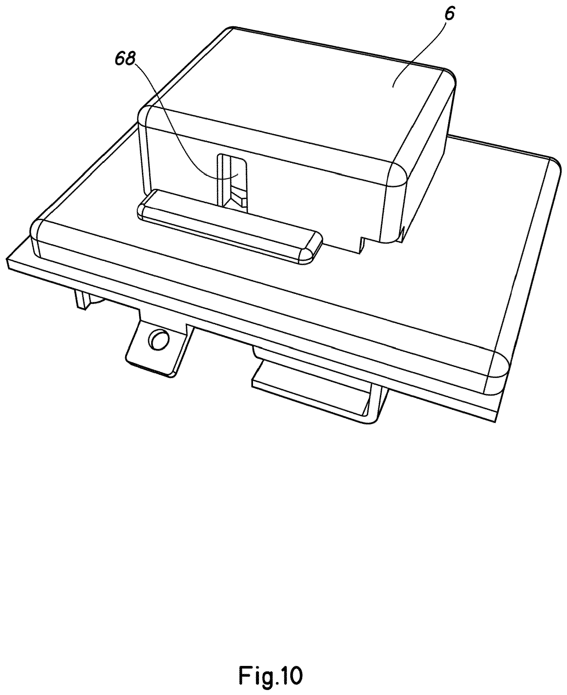

Fig. 4 to 10 show a first embodiment of a control device -2- according to the present invention.

In said embodiment, the device -2- consists of a case which, in addition to the screen -22- and the warning light -21- mentioned above, also has internally an ethylene sensor -3-, an electricity supply -5- and an electronic control unit -4- (for example, a printed circuit), the main function of which is to control the means for access of outside air to the sensor -3-, by opening and closing said means, and which in this case also controls the operation of the screen -22- and the warning light -21-.

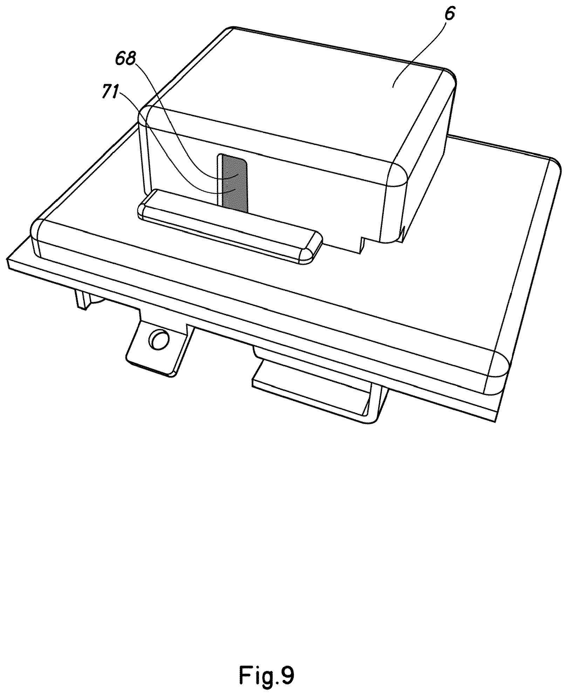

The means for access of outside air to the sensor comprise, in this case a gate -6- operated by a motor -8-. In the embodiment in Fig. 4 to 10, the motor -8- is a coil which moves an actuator -7-. The actuator -7- runs along a groove -72-, causing an interposition part -71- to close (by filling) or open an access aperture -68- situated on the gate -6-. Closing the access aperture -88- occurs when the interposition part coincides with the aperture -68- (see Fig. 9). Although not shown, the interposition part and/or the aperture may have joints to provide a hermetic seal.

In this case, the gate is fixed. In this case also the gate houses the measurement portion of the sensor -3-. A spring facilitates the return of the actuator. The gate -6- and access aperture -88- thereof are situated in the lower portion of the module. In this case, the gate -6- performs the functions of housing the sensor, although gate and housing could be separate entities. In addition, in the case shown, the gate -6- projects relative to the rest of the control device -2-.

In this case, the control -4- opens and closes the access of the gate -6- selectively by means of the actuator -7-. The sensor therefore only works periodically, not continuously. Although not shown in the figures, the control device -2- may have means for carrying out cleaning/maintenance tasks on the sensor, in order to extend further the useful life thereof.

The casing of the control device -2- will preferably be sealed, in order to protect the sensor -3- from the outside air including when not taking measurements.

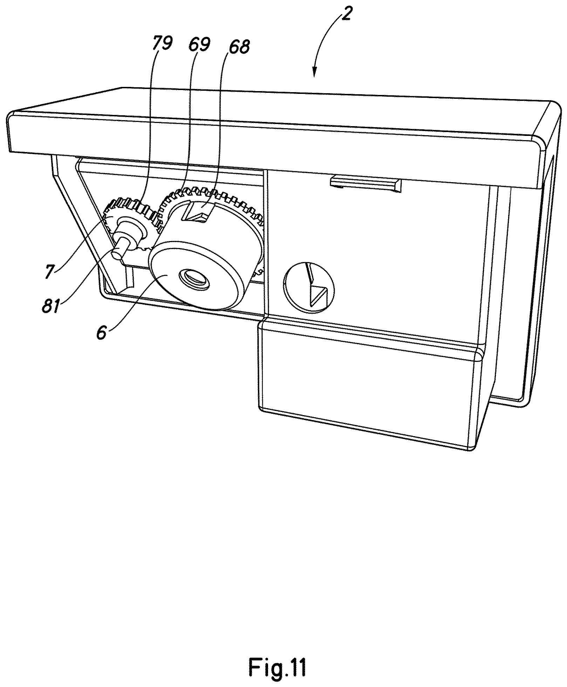

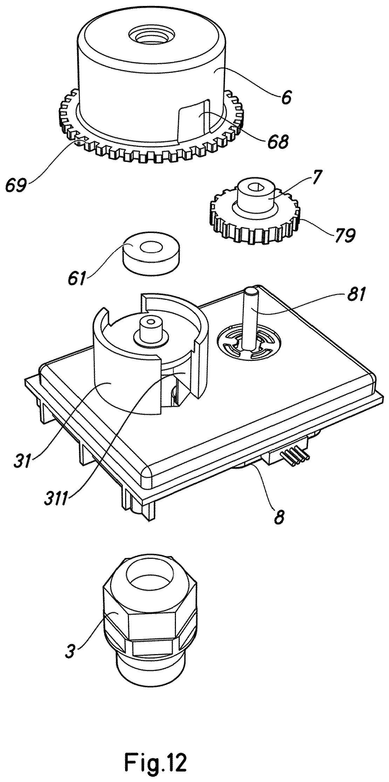

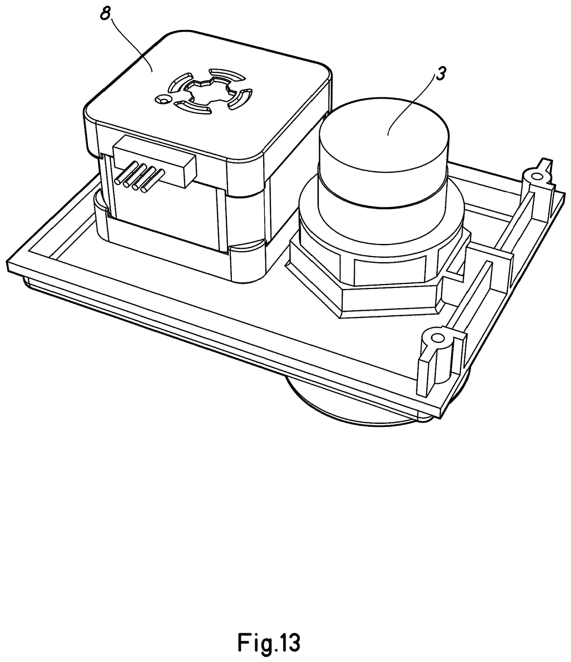

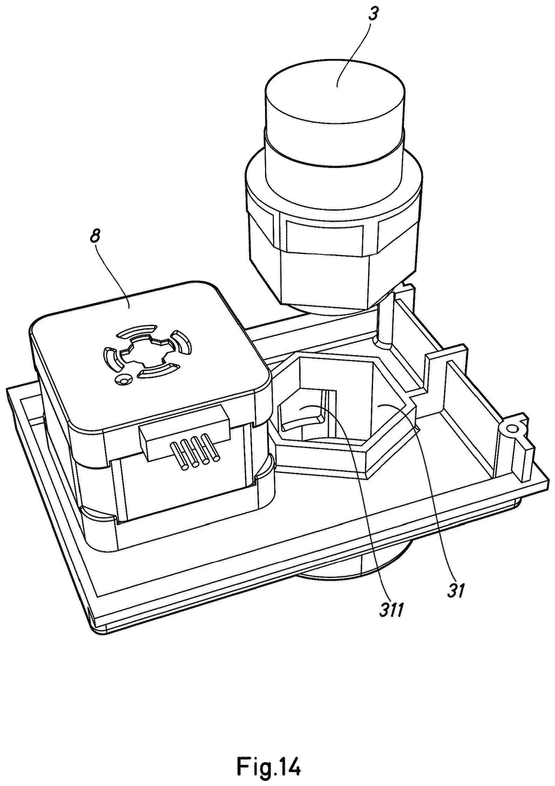

In Fig. 11 to 16 a second embodiment of the ethylene sensor control device -2- according to the present invention is shown. Elements that are the same, similar or equivalent have been identified with identical reference numerals. Said elements will therefore not be explained in detail.

Whereas in the embodiment in Fig. 4 to 10 actuation of the gate is linear, in the embodiment in Fig. 11 to 16, actuation of the gate is rotary. In the example shown, a stepper motor -8- is used for this purpose, although other types of motor or mini motor are also possible.

In this case, actuation of the gate is rotary. The motor -8- actuates a shaft -81- rotationally, which transmits the rotary movement to an actuator -7- which comprises a toothed gear wheel -79- for transmitting the movement to another toothed gear wheel -69- which forms part of the gate -6-. In this case, the gate -6- and the aperture thereof -68- are moveable and also act as a shutter. The actuator -7- and the gate -6- are situated on the outer portion of the device -2-, and are intended to be located inside an ethylene absorption module.

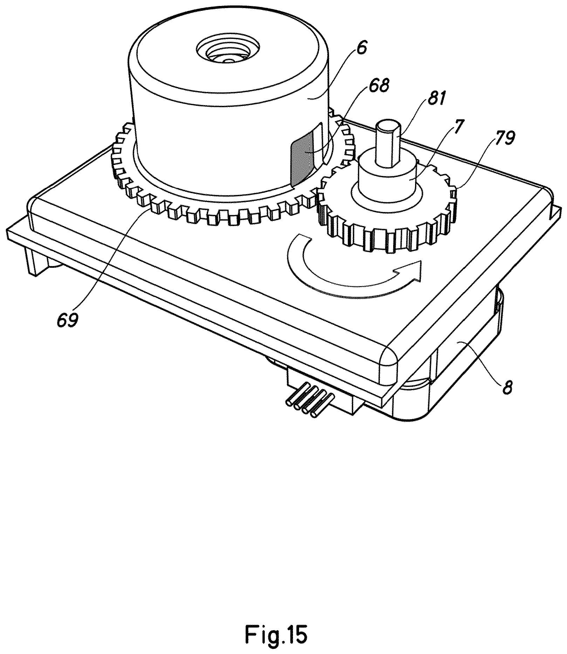

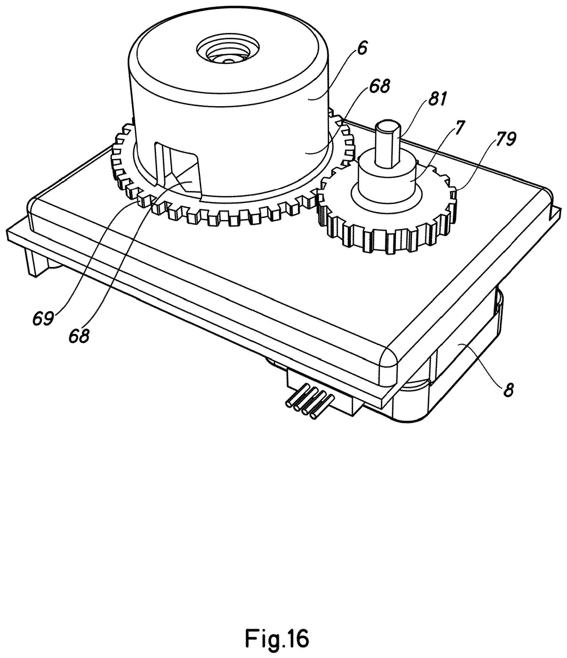

In this case, the sensor -3- is situated in a special housing -31- situated inside the gate -6-. The housing -31- has an aperture -311- giving access to the sensor. The outer casing can rotate relative to the housing -31- until the respective apertures -68-, -311- of the gate -6- and of the housing -31- coincide, allowing air from the module to reach the sensor -3- for measurement. The relative rotation between the gate and the housing is facilitated by a bearing -61-. Fig. 15 and 16 show the passage from the closed position to the open position for reading the ethylene concentration. In the closed position, the aperture -68- of the gate coincides with the walls of the housing -31-, which closes off the entry of air from outside the control device (the interior space of the module).

As it is not necessary for the sensor to operate continuously, and as the sensor can be protected from particles, increases in humidity and ethylene concentrations, the lifespan of the ethylene sensor is extended. Although not shown in the figures, the control device may have automatic cleaning and/or maintenance means for the sensor in order to prolong the life thereof.

Although the invention has been presented and described with reference to embodiments thereof, it will be understood that said embodiments do not limit the invention, and many structural or other details which will be evident to persons skilled in the art after interpreting the subject matter disclosed in the present description, claims and drawings may vary. Thus all variants and equivalents will fall within the scope of the present invention if said variants or equivalents can be considered to fall within the most extensive scope of the following claims.

## Claims

1. Ethylene-absorbing module (1) for fruit conservation, which comprises an outer casing and a space for receiving gas filters, ethylene filters or ethylene absorbing material, and a gas sensor control device (2) for fruit, vegetable and/or flower conservation installations, **characterised in that** the gas sensor control device (2) comprises a closed casing with a gate (6) that can be actuated, a gas sensor (3) arranged inside the casing, and the gate (6) giving access to the measurement area of the sensor (3), the device (2) also comprising automatic control means for the selective opening of the gate (6) so that the sensor (3) can measure gas concentration.

2. Module (1) according to the preceding claim, **characterised in that** the closed casing of the gas sensor control device (2) is sealed.

3. Module (1) according to any of the preceding claims, **characterised in that** the automatic control means of the gas sensor control device (2) comprise a programmable electronic circuit.

4. Module (1) according to any of the preceding claims, **characterised in that** the gas sensor control device (2) also comprises a screen (22) configured to show the result of a measurement taken by the sensor.

5. Module (1) according to any of the preceding claims, **characterised in that** the gas sensor control device (2) also comprises a warning light (21).

6. Module (1) according to any of the preceding claims, **characterised in that** the automatic control means of the gas sensor control device (2) actuate a motor (8) which in turn actuates an actuator (7) for the gate (6).

7. Module (1) according to the preceding claim, **characterised in that** the actuator (7) slides along a groove in order to close/open an aperture in the gate (6) using a closing part.

8. Module (1) according to claim 6, **characterised in that** the actuator (7) causes the gate (6) to rotate so that an aperture in the gate (6) coincides with the walls or an aperture of a housing for the gas sensor (3).

9. Module (1) according to any of the preceding claims, **characterised in that** the gate (6) of the gas sensor control device (2) is situated inside the module (1).

10. Module (1) according to any of the preceding claims, **characterised in that** the module (1) comprises means for forcing air to pass through a space intended to receive air filters, the device being situated between said space and an outlet in the module (1) for the forced air.

11. Module (1) according to the preceding claim, **characterised in that** the means for forcing the passage of the air comprises at least one ventilator.

12. Module (1) according to either claim 10 or claim 11, **characterised in that** it comprises a filter for solids situated between the space for receiving gas filters and the gas sensor control device (2).

13. Module (1) according to any of the preceding claims, **characterised in that** said outer casing comprises wheels for the transport thereof.

## Patentansprüche

1. Ethylenabsorbierendes Modul (1) für die Obstkonservierung, das ein äußeres Gehäuse und einen Raum zur Aufnahme von Gasfiltern, Ethylenfiltern oder ethylenabsorbierendem Material sowie eine Gassensor-Steuereinrichtung (2) für Obst-, Gemüse- und/oder Blumenkonservierungsanlagen umfasst, **dadurch gekennzeichnet, dass** die Gassensor-Steuervorrichtung (2) ein geschlossenes Gehäuse mit einer betätigbaren Klappe (6), einen im Inneren des Gehäuses angeordneten Gassensor (3) und die Klappe (6), die den Zugang zum Messbereich des Sensors (3) ermöglicht, umfasst, wobei die Vorrichtung (2) auch automatische Steuermittel für die selektive Öffnung der Klappe (6) umfasst, damit der Sensor (3) die Gaskonzentration messen kann.

2. Modul (1) nach dem vorhergehenden Anspruch, **dadurch gekennzeichnet, dass** das geschlossene Gehäuse der Gassensor-Steuervorrichtung (2) abgedichtet ist.

3. Modul (1) nach einem der vorhergehenden Ansprüche, **dadurch gekennzeichnet, dass** die automatischen Steuermittel der Gassensor-Steuervorrichtung (2) eine programmierbare elektronische Schaltung umfassen.

4. Modul (1) nach einem der vorhergehenden Ansprüche, **dadurch gekennzeichnet, dass** die Gassensor-Steuervorrichtung (2) auch einen Bildschirm (22) umfasst, der so konfiguriert ist, dass er das Ergebnis einer von dem Sensor vorgenommenen Messung anzeigt.

5. Modul (1) nach einem der vorhergehenden Ansprüche, **dadurch gekennzeichnet, dass** die Gassensor-Kontrolleinrichtung (2) auch eine Warnleuchte (21) umfasst.

6. Modul (1) nach einem der vorhergehenden Ansprüche, **dadurch gekennzeichnet, dass** die automatischen Steuermittel der Gassensor-Steuereinrichtung (2) einen Motor (8) betätigen, der seinerseits einen Aktuator (7) für die Klappe (6) betätigt.

7. Modul (1) nach dem vorhergehenden Anspruch, **dadurch gekennzeichnet, dass** der Aktuator (7) entlang einer Nut gleitet, um eine Öffnung der Klappe (6) mit Hilfe eines Schließteils zu schlie-ßen/öffnen.

8. Modul (1) nach Anspruch 6, **dadurch gekennzeichnet, dass** der Aktuator (7) die Klappe (6) in Drehung versetzt, so dass eine Öffnung in der Klappe (6) mit den Wänden oder einer Öffnung eines Gehäuses für den Gassensor (3) zusammenfällt.

9. Modul (1) nach einem der vorhergehenden Ansprüche, **dadurch gekennzeichnet, dass** die Klappe (6) der Gassensor-Steuereinrichtung (2) im Inneren des Moduls (1) angeordnet ist.

10. Modul (1) nach einem der vorhergehenden Ansprüche, **dadurch gekennzeichnet, dass** das Modul (1) Mittel zum Durchleiten von Luft durch einen Raum umfasst, der zur Aufnahme von Luftfiltern bestimmt ist, wobei die Vorrichtung zwischen dem genannten Raum und einem Auslass im Modul (1) für die durchgeleitete Luft angeordnet ist.

11. Modul (1) nach dem vorhergehenden Anspruch, **dadurch gekennzeichnet, dass** die Mittel zum Durchleiten des Durchgangs der Luft mindestens einen Ventilator umfassen.

12. Modul (1) nach Anspruch 10 oder 11, **dadurch gekennzeichnet, dass** es einen Feststofffilter umfasst, der sich zwischen dem Raum zur Aufnahme von Gasfiltern und der Gassensor-Steuervorrichtung (2) befindet.

13. Modul (1) nach einem der vorhergehenden Ansprüche, **dadurch gekennzeichnet, dass** das Außengehäuse Räder für seinen Transport umfasst.

## Revendications

1. Module absorbant de l'éthylène (1) pour une conservation de fruits, qui comprend un boîtier extérieur et un espace pour la réception de filtres à gaz, de filtres à éthylène ou d'un matériau absorbant de l'éthylène, et un dispositif de commande de capteur de gaz (2) pour des installations de conservation de fruits, de légumes et/ou de fleurs, **caractérisé en ce que** le dispositif de commande de capteur de gaz (2) comprend un boîtier fermé muni d'une porte (6) pouvant être actionnée, un capteur de gaz (3) disposé à l'intérieur du boîtier, et la porte (6) donnant accès à la zone de mesure du capteur (3), le dispositif (2) comprenant également des moyens de commande automatique pour l'ouverture sélective de la porte (6) de sorte que le capteur (3) peut mesurer une concentration en gaz.

2. Module (1) selon la revendication précédente, **caractérisé en ce que** le boîtier fermé du dispositif de commande de capteur de gaz (2) est fermé de manière étanche.

3. Module (1) selon l'une quelconque des revendications précédentes, **caractérisé en ce que** les moyens de commande automatique du dispositif de commande de capteur de gaz (2) comprennent un circuit électronique programmable.

4. Module (1) selon l'une quelconque des revendications précédentes, **caractérisé en ce que** le dispositif de commande de capteur de gaz (2) comprend également un écran (22) configuré pour afficher le résultat d'une mesure effectuée par le capteur.

5. Module (1) selon l'une quelconque des revendications précédentes, **caractérisé en ce que** le dispositif de commande de capteur de gaz (2) comprend également un témoin lumineux (21).

6. Module (1) selon l'une quelconque des revendications précédentes, **caractérisé en ce que** les moyens de commande automatique du dispositif de commande de capteur de gaz (2) actionnent un moteur (8) qui à son tour actionne un actionneur (7) pour la porte (6).

7. Module (1) selon la revendication précédente, **caractérisé en ce que** l'actionneur (7) glisse le long d'une rainure afin de fermer/ouvrir une ouverture dans la porte (6) à l'aide d'une partie de fermeture.

8. Module (1) selon la revendication 6, **caractérisé en ce que** l'actionneur (7) fait tourner la porte (6) de telle sorte qu'une ouverture dans la porte (6) coïncide avec les parois ou une ouverture d'un boîtier pour le capteur de gaz (3).

9. Module (1) selon l'une quelconque des revendications précédentes, **caractérisé en ce que** la porte (6) du dispositif de commande de capteur de gaz (2) est située à l'intérieur du module (1).

10. Module (1) selon l'une quelconque des revendications précédentes, **caractérisé en ce que** le module (1) comprend des moyens pour forcer de l'air à passer à travers un espace destiné à recevoir des filtres à air, le dispositif étant situé entre ledit espace et une sortie dans le module (1) pour l'air forcé.

11. Module (1) selon la revendication précédente, **caractérisé en ce que** les moyens pour forcer le passage de l'air comportent au moins un ventilateur.

12. Module (1) selon la revendication 10 ou la revendication 11, **caractérisé en ce qu'**il comprend un filtre pour solides situé entre l'espace de réception de filtres à gaz et le dispositif de commande de capteur de gaz (2).

13. Module (1) selon l'une quelconque des revendications précédentes, **caractérisé en ce que** ledit boîtier extérieur comprend des roues pour son transport.
